(19) Europäisches Patentamt
European Patent Office
Office européen des brevets

(11) **EP 3 548 879 B1**

(12) **FASCICULE DE BREVET EUROPEEN**

(45) Date de publication et mention de la délivrance du brevet:
**05.07.2023   Bulletin 2023/27**

(21) Numéro de dépôt: **17818454.5**

(22) Date de dépôt: **01.12.2017**

(51) Classification Internationale des Brevets (IPC):
*G01M 5/00* *(2006.01)*      *G01N 27/02* *(2006.01)*
*G01N 33/38* *(2006.01)*      *G01R 31/11* *(2006.01)*
*E04C 5/00* *(2006.01)*

(52) Classification Coopérative des Brevets (CPC):
**G01N 27/02; G01M 5/0033; G01M 5/0083;**
**G01N 33/383; G01R 31/11;** E04C 5/00

(86) Numéro de dépôt international:
**PCT/EP2017/081207**

(87) Numéro de publication internationale:
**WO 2018/100167 (07.06.2018 Gazette 2018/23)**

(54) **PROCÉDÉ DE SURVEILLANCE D'UN OUVRAGE DE GÉNIE CIVIL**

VERFAHREN ZUR ÜBERWACHUNG EINES BAUWERKS

METHOD FOR MONITORING A CIVIL ENGINEERING CONSTRUCTION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **01.12.2016   FR 1661786**

(43) Date de publication de la demande:
**09.10.2019   Bulletin 2019/41**

(73) Titulaire: **Electricité de France**
**75008 Paris (FR)**

(72) Inventeur: **TAILLADE, Frédéric**
**92140 Clamart (FR)**

(74) Mandataire: **Regimbeau**
**20, rue de Chazelles**
**75847 Paris Cedex 17 (FR)**

(56) Documents cités:
**US-A1- 2016 146 697**

• **NAOSHI HIRAI ET AL: "Highly sensitive detection of distorted points in a cable by frequency domain reflectometry", PROCEEDINGS OF 2014 INTERNATIONAL SYMPOSIUM ON ELECTRICAL INSULATING MATERIALS, 1 juin 2014 (2014-06-01), pages 144-147, XP055375229, DOI: 10.1109/ISEIM.2014.6870741 ISBN: 978-4-88686-086-6**
• **OHKI YOSHIMICHI ET AL: "Highly sensitive location method of an abnormal temperature point in a cable by frequency domain reflectometry", 2013 IEEE INTERNATIONAL CONFERENCE ON SOLID DIELECTRICS (ICSD), IEEE, 30 juin 2013 (2013-06-30), pages 117-120, XP032496810, ISSN: 2159-1687, DOI: 10.1109/ICSD.2013.6619799 ISBN: 978-1-4799-0807-3 [extrait le 2013-10-03]**
• **MARK W. LIN ET AL: "Crack damage detection of concrete structures using distributed electrical time domain reflectometry (ETDR) sensors", SPIE - INTERNATIONAL SOCIETY FOR OPTICAL ENGINEERING. PROCEEDINGS, vol. 3671, 18 mai 1999 (1999-05-18), pages 297-304, XP055375344, US ISSN: 0277-786X, DOI: 10.1117/12.348680 ISBN: 978-1-5106-0753-8**
• **ZHI ZHOU ET AL: "Development of a Distributed Crack Sensor Using Coaxial Cable", SENSORS, vol. 16, no. 8, 29 juillet 2016 (2016-07-29), page 1198, XP055374340, CH ISSN: 1424-8220, DOI: 10.3390/s16081198**

- **SHISHUANG SUN ET AL: "A Novel TDR-Based Coaxial Cable Sensor for Crack/Strain Sensing in Reinforced Concrete Structures", IEEE TRANSACTIONS ON INSTRUMENTATION AND MEASUREMENT, IEEE SERVICE CENTER, PISCATAWAY, NJ, US, vol. 58, no. 8, 1 août 2009 (2009-08-01), pages 2714-2725, XP011263804, ISSN: 0018-9456**
- **Wei Liu ET AL: "AN OVERVIEW OF CORROSION DAMAGE DETECTION IN STEEL BRIDGE STRANDS USING TDR", , 31 December 2001 (2001-12-31), pages 1-9, XP055576257, Retrieved from the Internet: URL:https://www.semanticscholar.org/paper/ AN-OVERVIEW-OF-CORROSION-DAMAGE-DET ECTION- IN-STEEL-Liu-Hunsperger/cb654adf3653440567 46181b60c71d8b396662f1 [retrieved on 2019-04-01]**

**Description**

DOMAINE TECHNIQUE GENERAL

L'invention concerne la surveillance des structures du type ouvrages de génie civil (nucléaire, hydraulique, routier, etc.) ou encore éoliennes et en particulier celles comportant des armatures de renfort métalliques.

ETAT DE LA TECHNIQUE

L'usage d'armatures métalliques est très courant dans les ouvrages de génie civil. Par exemple, on utilise des câbles métalliques sous contraintes pour améliorer les propriétés mécaniques de certains ouvrages en béton. Un autre exemple consiste à placer des bandes métalliques ou treillis métalliques dans des ouvrages de type sol renforcé. Encore un autre exemple consiste à placer dans une gaine (précontrainte) des câbles en acier injecté dans certain cas au coulis de ciment ou à la cire pétrolifère pour maintenir ou contraindre une structure ou un bâtiment. Encore un autre exemple consiste à utiliser des tirants d'ancrage métalliques pour maintenir ou contraindre une structure ou un bâtiment.

Les armatures métalliques en acier présentent de très bonnes propriétés mécaniques mais ont pour inconvénient d'être soumises aux attaques de la corrosion. Il est connu de protéger de telles armatures métalliques en acier par une galvanisation, par une métallisation, par une protection cathodique ou par une autre méthode de passivation ou de gainage. Cependant, il apparaît nécessaire de surveiller, au cours du temps, l'état de santé de telles armatures métalliques, notamment pour les ouvrages dont la durée d'utilisation peut dépasser plusieurs dizaines d'années. Il est préférable de pouvoir procéder à une telle surveillance de manière non intrusive, c'est-à-dire sans intervenir directement dans le corps de l'ouvrage.

Les articles

"Crack damage détection of concrète structures using distributed electrical time domain reflectometry (ETDR) sensors", MARK W. LIN ET AL, SPIE - INTERNATIONAL SOCIETY FOR OPTICAL ENGINEERING, vol. 3671, 18 mai 1999, pages 297-304,

"A Novel TDR-Based Coaxial Cable Sensor for Crack/Strain Sensing in Reinforced Concrete Structures", SHISHUANG SUN ET AL, IEEE TRANSACTIONS ON INSTRUMENTATION AND MEASUREMENT, vol. 58, no. 8, 1 août 2009, pages 2714-2725, et

"An Overview of Corrosion Damage Détection in Steel Bridge Strands Using TDR", WEI LIU ET AL, 31 décembre 2001, pages 1-9,

décrivent des procédés de surveillance de structures en utilisant des câbles.

Il existe cependant un besoin d'améliorer les solutions non intrusives connues pour surveiller l'état de santé des armatures métalliques et pouvoir détecter un défaut structurel éventuel lié à la corrosion et/ou au vieillissement par exemple

PRESENTATION DE L'INVENTION

L'invention propose de pallier au moins un de ces inconvénients.

A cet effet, l'invention propose un procédé selon la revendication indépendante 1 pour surveiller un ouvrage de génie civil comprenant au moins une première armature métallique contribuant à la tenue mécanique dudit ouvrage, et un câble électrique constitué d'au moins une paire de conducteurs électriques ayant une première extrémité électriquement accessible, le procédé comprenant les étapes suivantes :

   a) mesure du coefficient de réflexion en impédance du câble électrique en fonction de la fréquence ;
   b) traitement des coefficients de réflexions S11 récupérés pour en déduire l'impédance caractéristique Zc du câble électrique en fonction de la distance ;
   c) comparaison de l'impédance caractéristique avec une impédance de référence Z0 correspondant à un état de référence du câble électrique de manière à en déduire une variation d'impédance $\Delta Z$ le long du câble électrique fonction d'une grandeur physique caractéristique du câble.

L'étape b) selon la revendication indépendante 1 comprend des sous-étapes b1) à b5) qui sont détaillées ci-après.

L'invention est avantageusement complétée par les caractéristiques suivantes, prises seules ou en une quelconque de leur combinaison techniquement possible.

La grandeur physique caractéristique du câble est une déformation $\varepsilon_z$ longitudinale du câble électrique, la

variation d'impédance $\Delta Z$ étant définie par $\Delta Z = C_\varepsilon \times \varepsilon_z$ avec $C_\varepsilon$ la sensibilité à la déformation du câble électrique qui dépend, de préférence, des caractéristiques géométriques et des matériaux du câble électrique.

La grandeur physique caractéristique du câble électrique est une variation de température fonction du coefficient de sensibilité à la température ou une pression radiale, la variation d'impédance $\Delta Z$ étant définie par $\Delta Z = C_t \times \Delta t$ avec $C_t$ la sensibilité à la température et $\Delta t$ la variation de température.

Le procédé comprend une étape de détermination d'une impédance de référence du câble électrique, ledit câble électrique, dans cet état de référence, étant pris isolément et indépendamment de l'ouvrage de génie civil à surveiller.

La détermination de l'impédance de référence comprend les étapes a), b), c), l'étape c) de traitement permettant d'obtenir ladite impédance de référence Z0.

La mesure du coefficient de réflexion est mise en oeuvre au moyen d'un analyseur de réseau.

L'invention concerne également un système de surveillance d'un ouvrage de génie civil, l'ouvrage comprenant au moins une première armature métallique contribuant à la tenue mécanique dudit ouvrage, et un câble électrique constitué d'au moins une paire de conducteurs électriques ayant une première extrémité électriquement accessible, le système comprenant une unité de traitement configurée pour mettre en oeuvre un procédé selon l'invention.

L'invention permet de réaliser une mesure distribuée de la déformation le long d'un conducteur électrique introduit dans l'ouvrage avant injection du coulis de ciment.

Ainsi, une rupture d'armature(s) engendre une déformation anormale du coulis au voisinage immédiat de la rupture qui est mesurée par l'invention.

La solution de l'invention est économique puisqu'elle nécessite une instrumentation au moins 10 à 20 fois moins chère que d'autres solutions existantes reposant sur la mesure de déformation par fibre optique.

Elle est également compétitive sur les projets neufs puisque cette solution permet d'éviter une inflation de l'instrumentation classique reposant sur des mesures de déformation ponctuelle (témoin sonore) qui par ailleurs ne fournit pas une réponse satisfaisante, tout en améliorant l'information en détection et localisation des ruptures.

PRESENTATION DES FIGURES

D'autres caractéristiques, buts et avantages de l'invention ressortiront de la description qui suit, qui est purement illustrative et non limitative, et qui doit être lue en regard des dessins annexés sur lesquels :

- la figure 1 illustre un ouvrage d'art surveiller selon l'invention ;
- la figure 2 illustre des étapes d'un procédé de surveillance selon l'invention ;
- la figure 3 illustre une vue générale d'un câble coaxial ;
- la figure 4 illustre une vue en coupe d'un câble coaxial ;
- les figures 5a et 5b illustrent le coefficient de réflexion en fonction de la fréquence (module et phase) ;
- la figure 6 illustre l'impédance caractéristique en fonction de la position dans l'état de référence et l'état déformé ;
- la figure 7 illustre la variation d'impédance caractéristique entre l'état de référence et l'état déformé ;
- la figure 8 illustre la variation d'impédance caractéristique entre un état sollicité en température et éventuellement en déformation et un état de référence.

Sur l'ensemble des figures les éléments similaires portent des références identiques.

DESCRIPTION DETAILLEE DE L'INVENTION

*Système et procédé de mesure*

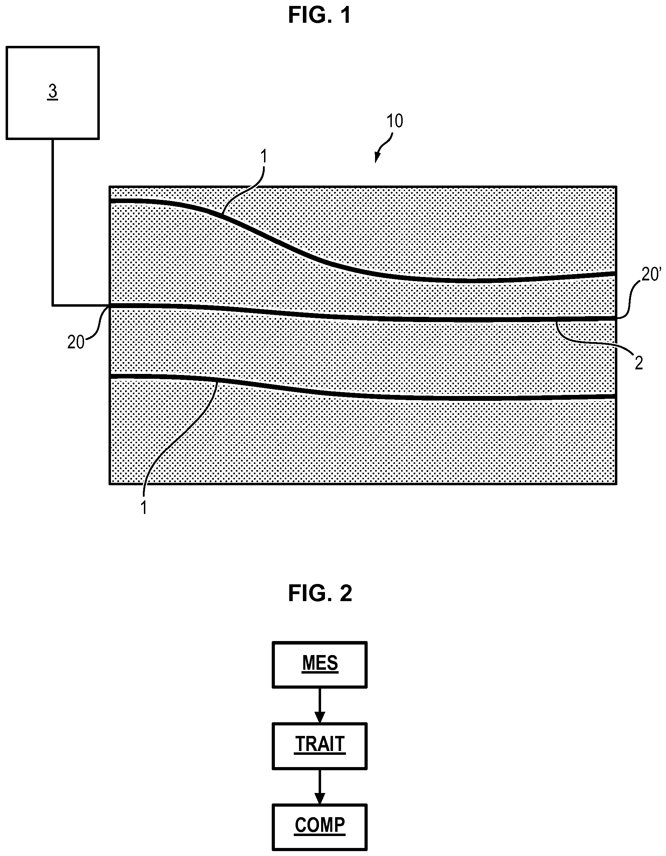

La **figure 1** illustre un ouvrage de génie civil 10 renforcé par au moins une armature 1 métallique telle que connue.

Ce type d'ouvrage de génie civil peut être un ouvrage en béton armé avec ou sans précontrainte pour des applications dans les domaines nucléaire, hydraulique, ouvrage d'art (pont, viaduc), une fondation d'éolienne ou bien en géomatériau pour des applications concernant les sols, les digues.

Les armatures 1 sont classiquement protégées de la corrosion en fonction de l'application et de la réalisation : acier galvanisé pour les armatures de terre armée, coulis de ciment et/ou cire pétrolifère pour la précontrainte.

Pour s'assurer du bon état des armatures 1 dans l'ouvrage, on procède à une surveillance de l'ouvrage selon un procédé ci-dessous décrit en relation avec la **figure 2** et mis en oeuvre par une unité de traitement telle qu'un processeur (non représenté).

A nouveau en relation avec la figure 1, outre les armatures 1, l'ouvrage comprend un câble électrique 2 constitué d'au moins deux éléments conducteurs, le câble ayant une première extrémité 20 et une seconde extrémité 20'.

Ce câble électrique 2 a été introduit dans l'ouvrage à des fins de surveillance de ce dernier et est noyé dans le coulis de ciment de l'ouvrage typiquement dans le cas de la précontrainte. Le câble électrique 2 est constitué d'au moins deux conducteurs électriques est par exemple un câble coaxial ou une paire torsadée. Certaines caractéristiques du câble électrique 2 sont connus (matériau, diamètre, etc.).

Afin de surveiller l'ouvrage, on procède à la mesure MES du coefficient de réflexion en impédance S11 du câble électrique 2

Une telle mesure est mise en oeuvre au moyen d'un analyseur 3 de réseau (en anglais, « *Vector Network Analyser* », (VNA)) en liaison avec l'unité de traitement.

A partir de cette mesure, on obtient, par traitement numérique du signal, la distribution de l'impédance caractéristique du câble électrique2 en fonction du temps.

Connaissant l'impédance caractéristique et la structure du câble électrique, il est possible d'estimer la vitesse, dans le câble électrique, des ondes électriques ou de la supposer connues et donc d'exprimer la distribution d'impédance caractéristique du câble électrique 2 en fonction de la distance.

Dans le procédé selon l'invention, le traitement TRAIT numérique du signal (étape b)) consiste à :

- b1) extrapoler la réponse fréquentielle du câble électrique à une fréquence nulle afin de constituer le signal S11*,
- b2) appliquer un filtre passe bas (*FiltrePB(f)*) anti-repliement au signal S11* à l'aide d'un filtre passe bas de type Hanning, Hamming, Kaiser-Bessel, Flap-Top, etc,
- b3) calculer la transformée de Fourier inverse du signal S11* filtré en considérant le caractère causal et réel de la solution, c'est-à-dire qu'il ne peut y avoir de signal que pour des temps supérieur ou égale à 0 et que la solution est réelle ; on obtient donc la réponse impulsionnelle du câble électrique:

$$h(t) = TF^{-1}(S11^*(f) \times FiltrePB(f))$$

- b4) intégrer la réponse impulsionnelle, pour obtenir la réponse à un échelon

$$u(t) = \int_0^t h(\tau)d\tau$$

- b5) appliquer une conversion affine à u(t) pour transformer la réponse à un échelon en impédance :

$$Z_C(t) = Z_{VNA}\frac{1+u(t)}{1-u(t)}$$ avec $Z_{VNA}$ l'impédance interne du VNA (en général 50Ω).

L'intérêt de mesurer le coefficient de réflexion en fréquence est que le rapport signal à bruit de la mesure est meilleur qu'en temporel (mesure de la réflexion d'une impulsion électrique en temporel puis transformation de Fourier des mesures).

Ensuite, on compare COMP l'impédance caractéristique avec une impédance de référence Z0 correspondant à un état de référence du câble électrique de manière à en déduire une variation d'impédance ΔZ le long du câble électrique, fonction d'une grandeur physique caractéristique du câble.

L'état de référence du câble électrique est pris dans un contexte d'état de sollicitation initial de la structure à surveiller par exemple en température et/ou en déformation. La mesure se faisant par rapport à cet état initial pris pour référence.

Une grandeur physique est par exemple une déformation $\varepsilon_z$ longitudinale du câble électrique, la variation d'impédance ΔZ étant définie par $\Delta Z = C_\varepsilon \times \varepsilon_z$ avec $C_\varepsilon$ la sensibilité à la déformation du câble électrique.

Une autre grandeur physique est par exemple la température. Dans ce cas, la variation d'impédance ΔZ est définie par $\Delta Z = C_t \times \Delta t$ avec $C_t$ la sensibilité à la température et Δt la variation de température.

*Sensibilité à la déformation d'un câble coaxial*

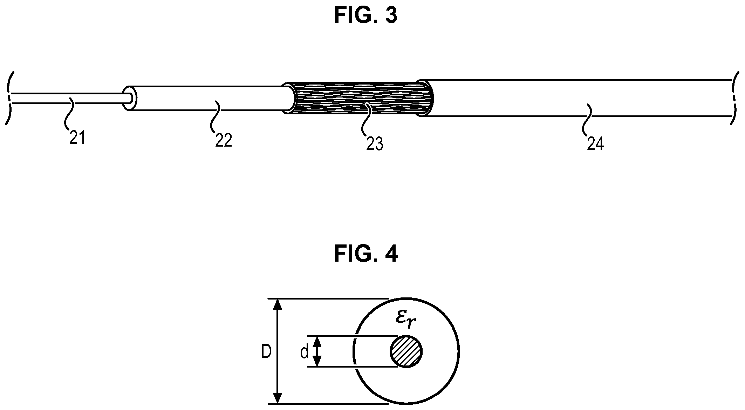

De préférence, le câble électrique est un câble coaxial. De manière connue et tel que cela est illustré sur la **figure 3** (et en coupe sur la **figure 4**), un tel câble est constitué d'une gaine de protection 24, d'une tresse métallique 23, d'un diélectrique 22 séparant la tresse de l'âme métallique 21.

L'impédance caractéristique $Z_C$ du câble coaxial et la vitesse de propagation $v_c$ d'un signal électrique dépendent de la géométrie de la section du câble et de la permittivité électrique $\varepsilon_r$ du matériau diélectrique :

$$Z_c = \sqrt{\frac{L}{C}} \text{ et } v_c = 1/\sqrt{LC}$$

avec $L$ (*H/m*) et $C$ (*F/m*) respectivement l'inductance et la capacité linéiques du câble coaxial, où $L = \frac{\mu_0 \mu_r}{2\pi} ln\left(\frac{D}{d}\right)$

et $C = 2\pi \varepsilon_0 \varepsilon_r / ln\left(\frac{D}{d}\right)$.

On obtient alors : $Z_c = \frac{ln\left(\frac{D}{d}\right)}{2\pi} \sqrt{\frac{\mu_0 \mu_r}{\varepsilon_0 \varepsilon_r}} = Z_0/\sqrt{\varepsilon_r}$ et $v_c = c/\sqrt{\varepsilon_r}$ où c représente la vitesse d'une onde électromagnétique dans le vide.

Pour un câble coaxial classique RG58 :

- l'impédance caractéristique vaut $Z_C$ = 50 $\Omega$
- la vitesse vaut $v_c = c \times 0,66$.

Lorsqu'on applique une contrainte au câble coaxial, qu'elle soit mécanique ou thermique, la géométrie de la section du câble et/ou la permittivité électrique du matériau diélectrique sont modifiées entraînant une variation d'impédance et/ou de vitesse caractéristique.

Dans le cas d'une déformation longitudinale $\varepsilon_z$ du câble coaxial, seule la géométrie de la section du câble coaxial varie par effet poisson entraînant une variation d'impédance caractéristique de $\Delta Z$ ; la vitesse reste inchangée. Un calcul de résistance des matériaux (RdM) donne le bon ordre de grandeur de la sensibilité à la déformation du câble coaxial $C_\varepsilon$, telle que la variation d'impédance s'écrive $\Delta Z = C_\varepsilon \times \varepsilon_z$.

En considérant les sections et les propriétés mécaniques (module d'Young et coefficient de poisson) de chaque matériau constituant le câble coaxial, nous pouvons calculer le module d'Young effectif du câble coaxial :

$$E_{eff} = \frac{1}{S_{total}} \frac{1}{\dfrac{1}{S_g E_g} + \dfrac{1}{S_d E_d} + \dfrac{1}{S_a E_a}}$$

avec la surface totale de la section du câble $S_{total} = \pi/4 \, d_g^2$, la surface de gaine $S_g = \pi/4 \left(d_g^2 - d_t^2\right)$, la surface du diélectrique $S_d = \pi/4 \left(d_d^2 - d_a^2\right)$ et la surface de l'âme centrale $S_a = \pi/4 \, d_a^2$, où $E_g$, $E_d$, $E_a$ sont les modules d'Young respectivement de la gaine, du diélectrique et de l'âme et où $d_g$, $d_t$, $d_d$, $d_a$ sont les diamètres extérieurs respectivement de la gaine, de la tresse, du diélectrique et de l'âme. On considère ici que le raideur de la tresse est négligeable devant celles des autres matériaux.

La déformation longitudinale du câble coaxial est donnée par $\varepsilon_z = \sigma_z/E_{eff}$ avec $\sigma_z$ la contrainte mécanique appliquée sur la section du câble coaxial. Par effet poisson, la déformation longitudinale $\varepsilon_z$ entraîne une déformation radiale de la section du câble coaxial notamment du matériau diélectrique $\varepsilon_{rd} = \sigma_d/E_d \times v_d$ et de l'âme $\varepsilon_{ra} = \sigma_a/E_a \times v_a$. Cette variation des diamètres se répercute alors sur l'impédance caractéristique du câble coaxial. On montre que cette variation d'impédance s'écrit alors :

$$\Delta Z = \frac{1}{2\pi} \sqrt{\frac{\mu_0 \mu_r}{\varepsilon_0 \varepsilon_r}} \left[ ln\left(\frac{d_d}{d_a}\frac{(1-\epsilon_{rd})}{(1-\epsilon_{ra})}\right) - ln\left(\frac{d_d}{d_a}\right) \right]$$

Ainsi, la sensibilité à la déformation du câble coaxial $C_\varepsilon = \Delta Z/\varepsilon_z$.

La sensibilité à la déformation du câble dépend des caractéristiques géométriques des câbles et la nature des matériaux utilisés. Pour un câble coaxial RG58, on a les propriétés suivantes :

- la gaine est réalisée en PVC ($E_g$ = 300 *MPa* ; $v_g$ = 0,4 ; $d_g$ = 4,95 *mm*)

- la tresse est réalisée en cuivre ($d_t$ = 3,45 *mm*)
- le diélectrique est réalisée en polyéthylène ($E_d$ = 800 *MPa* ; $v_d$ = 0,4 ; $d_d$ = 2,95 *mm* ; $\varepsilon_r$ = 2,35)
- l'âme est constituée de 19 brins de cuivre de diamètre 0,18 mm soit un diamètre constitué d'environ 6 brins ($E_a$ = 128 *GPa* ; $v_a$ = 0,3 ; $d_a$ = 1,08 *mm*)

Pour un tel câble, on trouve numériquement une sensibilité à la déformation $C_\varepsilon$ = -5,8 $\times$ 10$^{-6}$ $\Omega/(\mu m/m)$.

*Sensibilité à la température d'un câble coaxial*

En première approximation, la sensibilité à la température d'un câble coaxial provient de la dilatation des composants dans un plan de section du câble (figure 4). Sous l'effet de la chaleur, la gaine (tresse), le diélectrique et l'âme centrale se dilatent. Considérant que la tresse n'a pas de rigidité, la variation d'impédance peut s'exprimer en première approximation :

$$\Delta Z = \frac{1}{2\pi} \sqrt{\frac{\mu_0 \mu_r}{\varepsilon_0 \varepsilon_r}} \left[ \ln\left(\frac{d_d}{d_a}(1 + \alpha_T \Delta T)\right) - \ln\left(\frac{d_d}{d_a}\right) \right]$$

Avec $\alpha_T$ le coefficient de dilatation linéaire du diélectrique (pour le polyéthylène $\alpha_T$ = 2 $\times$ 10$^{-4}$ $K^{-1}$)

Pour un tel câble, on trouve numériquement une sensibilité à la température $C_T$ = +8 $\times$ 10$^{-3}$ $\Omega/K$.

Sous l'effet de la chaleur, les propriétés des matériaux constituant le câble électrique peuvent être modifiées ce qui contribuera à une variation d'impédance. Cette dépendance des propriétés matériaux à la température est mal connue mais pourra être estimée par un étalonnage en laboratoire.

De manière générale, les coefficients de sensibilité à la déformation, à la température ou à toute autre grandeur d'intérêt pourront être estimés par étalonnage en laboratoire.

*Expérimentation*

On a testé un câble coaxial pour vérifier la faisabilité de la mesure distribuée de déformation.

On a mesuré le coefficient de réflexion en impédance S11 d'un câble coaxial RG58 connecté à un analyseur de réseau (VNA Anritsu MS2026C). La mesure est réalisée dans une bande de fréquence située entre 2 MHz et 4 GHz par pas de 1 MHz.

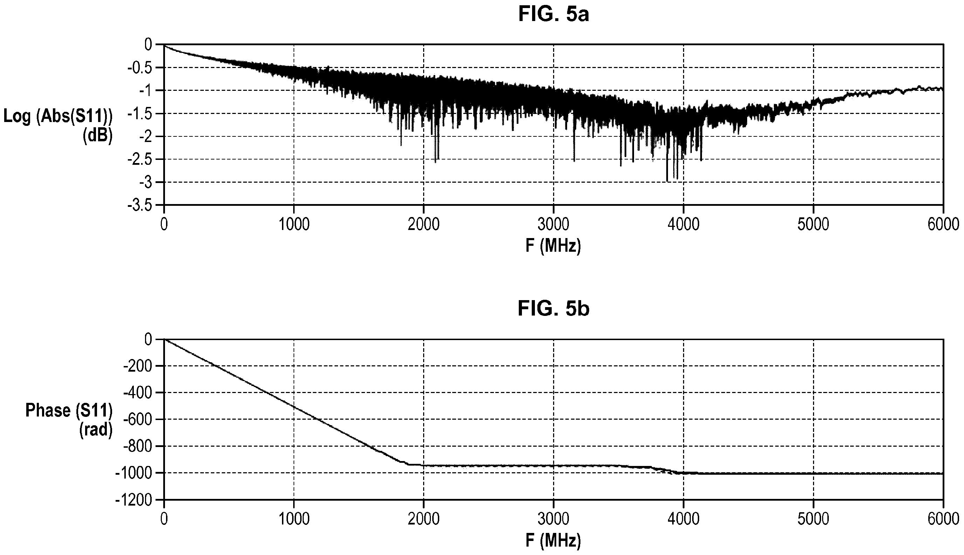

Le câble coaxial, d'une longueur d'environ 8 m, est attaché à une distance d'environ 5 m comptée à partir de l'extrémité du câble. Dans l'état de référence, une pré-charge est appliquée au câble grâce à une masse de 185 g suspendue 1 m plus loin que le point d'attache. Une mesure du coefficient de réflexion S11 est enregistrée dans cet état de référence : état 0. L'état déformé est réalisé en ajoutant, à la pré-charge, une masse de 740 g puis une mesure de S11 est enregistrée : état 1 (voir les **figures 5a et 5b**).

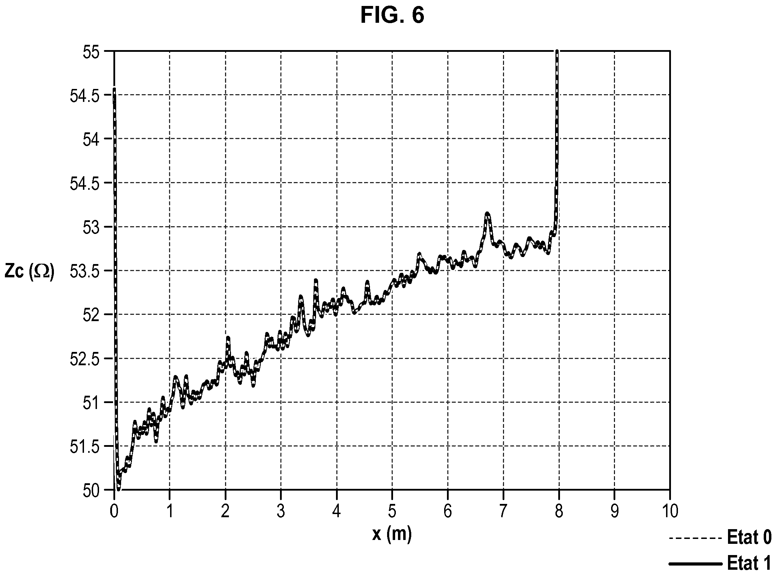

Les mesures du coefficient de réflexion S11 sont alors traitées selon notamment l'algorithme TRAIT pour déduire des mesures brutes, l'impédance caractéristique du câble coaxial en fonction de la position (voir **figure 6**). Tout autre algorithme pourrait être utilisé pour effectuer cette étape d'inversion. L'extrémité du câble située à environ 8 m est clairement observable sur la figure 6 correspondant à une impédance qui tend vers l'infini en théorie (impédance de l'extrémité du câble « open »).

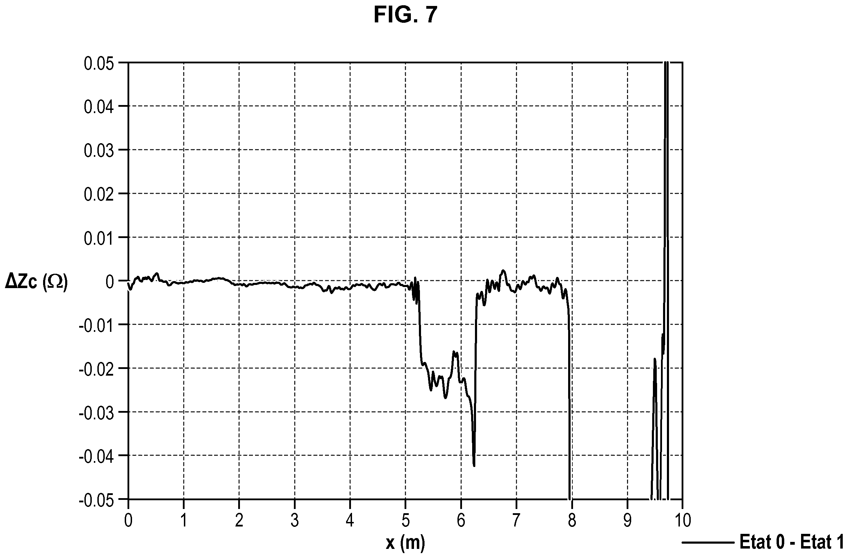

Après soustraction des impédances caractéristiques mesurées dans les états déformé et de référence, nous obtenons la variation d'impédance caractéristique répartie le long du câble coaxial (voir la **figure 7**).

Sur la figure 7, la zone en tension du câble coaxial apparaît clairement entre les abscisses 5,2 m et 6,2 m. Sur cette zone, l'amplitude de variation d'impédance est en moyenne de l'ordre de -0,0225 $\Omega$.

En reprenant les calculs théoriques présentés ci-dessus dans le cas d'une masse suspendue de 740 g, on obtient que la variation d'impédance théorique est égale à -0,0232 $\Omega$ ce qui est en bon accord avec la mesure expérimentale, compte tenu des incertitudes sur les valeurs des propriétés mécaniques du câble coaxial et des incertitudes de mesures.

On a ainsi montré la faisabilité de la méthode à réaliser une mesure distribuée de déformation le long d'un câble coaxial.

Sur le même principe, il est possible de faire une mesure distribuée de la température le long du câble coaxial. Comme dans l'exemple précédent, l'algorithme de traitement permet d'une part de transformer la mesure en fréquence en distribution d'impédance en fonction du temps ou de l'espace puis en calculant la différence d'impédance entre un état quelconque et un état de référence de mesurer l'évolution d'impédance le long du câble. Cette première partie est commune aux différentes applications. En fonction de l'application, un second traitement va traduire la différence d'impédance en variation de grandeur physique (température, déformation, etc.) grâce à un modèle d'interprétation de la mesure. Cela dépend donc de ce qui est recherché.

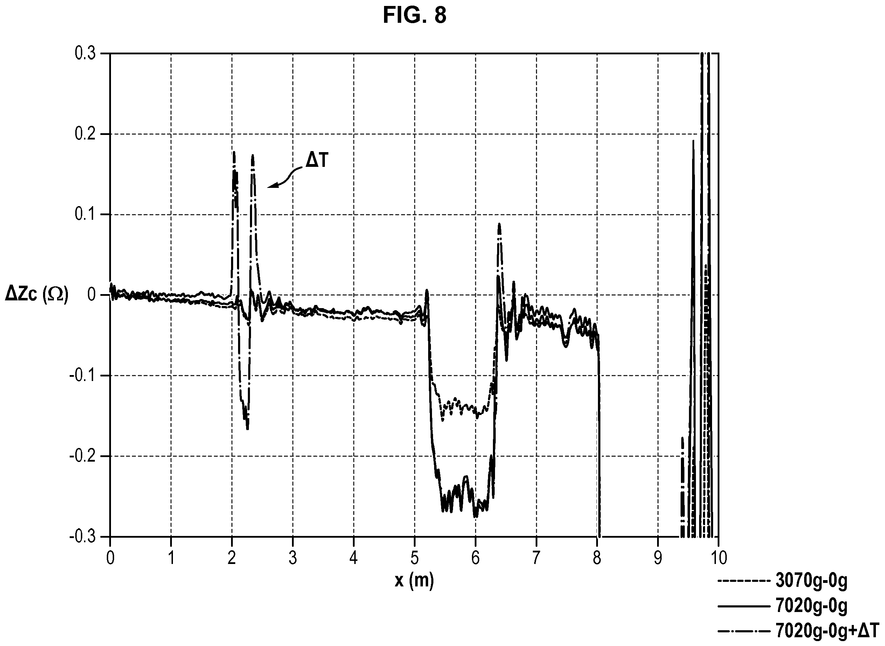

La **figure 8** montre une faisabilité d'une mesure distribuée de température imposée autour de l'abscisse 2,2 m le long d'un câble tout en continuant à mesurer la déformation. Notons au passage la linéarité de la mesure de déformation imposée grâce à des masses suspendues de 3070 g et 7020 g entre l'abscisse 5,2 m et 6,2 m. Le coefficient de sensibilité à la température est estimé expérimentalement à +0,013 Ω/°C. Compte tenu de l'essai de faisabilité, l'estimation du coefficient de sensibilité à la température est du bon ordre de grandeur de la valeur théorique (0,008 Ω/°C). L'estimation plus précise des coefficients de sensibilité peut se faire par étalonnage.

**Revendications**

1. Procédé pour surveiller un ouvrage de génie civil (10) comprenant au moins une première armature (1) métallique contribuant à la tenue mécanique dudit ouvrage, et un câble électrique (2) constitué d'au moins une paire de conducteurs électriques ayant une première extrémité (21) électriquement accessible, le procédé comprenant les étapes suivantes :

   a) mesure (MES), par un analyseur de réseau, du coefficient de réflexion en impédance (S11) du câble électrique en fonction de la fréquence ;
   b) traitement (TRAIT) des coefficients de réflexions S11 récupérés pour en déduire l'impédance caractéristique Zc du câble électrique en fonction de la distance comprenant les étapes suivantes :

      b1) extrapoler les coefficients de réflexion S11 à une fréquence nulle afin de constituer un signal S11* ;
      b2) appliquer un filtre passe bas anti-repliement au signal S11 * ;
      b3) calculer une transformée de Fourier inverse du signal S11* filtré en considérant que le signal S11* n'existe que pour des temps supérieurs ou égaux à 0 et qu'une solution du calcul de la transformée de Fourier inverse du signal S11* est réelle, de sorte à obtenir une réponse impulsionnelle du câble électrique : $h(t) = TF^{-1}(S11^*(f) \times FiltrePB(f))$, avec $TF^{-1}$ la transformée de Fourier inverse et $FiltrePB(f)$ le filtre passe-bas ;

      b4) intégrer la réponse impulsionnelle, pour obtenir une réponse à un échelon $u(t) = \int_0^t h(\tau)d\tau$ ; et
      b5) appliquer une conversion affine à la réponse à un échelon pour transformer la réponse à un échelon en une impédance : $Z_c(t) = Z_{VNA}\frac{1+u(t)}{1-u(t)}$ , avec $Z_{VNA}$ une impédance interne de l'analyseur de réseau ;

   c) comparaison (COMP) de l'impédance caractéristique avec une impédance de référence Z0 correspondant à un état de référence du câble électrique de manière à en déduire une variation d'impédance $\Delta Z$ le long du câble électrique fonction d'une grandeur physique caractéristique du câble.

2. Procédé selon la revendication 1, dans lequel la grandeur physique caractéristique du câble est une déformation $\varepsilon_z$ longitudinale du câble électrique, la variation d'impédance $\Delta Z$ étant définie par $\Delta Z = C_\varepsilon \times \varepsilon_z$ avec $C_\varepsilon$ la sensibilité à la déformation du câble électrique qui dépend, de préférence, des caractéristiques géométriques et des matériaux du câble électrique (2).

3. Procédé selon la revendication 1, dans lequel la grandeur physique caractéristique du câble électrique est une variation de température fonction du coefficient de sensibilité à la température ou une pression radiale, la variation d'impédance $\Delta Z$ étant définie par $\Delta Z = C_t \times \Delta t$ avec $C_t$ la sensibilité à la température et $\Delta t$ la variation de température.

4. Procédé selon l'une des revendications 1 à 3, comprenant une étape de détermination d'une impédance de référence du câble électrique (2), ledit câble électrique, dans cet état de référence, étant pris isolément et indépendamment de l'ouvrage de génie civil (10) à surveiller.

5. Procédé selon la revendication 4, dans lequel la détermination de l'impédance de référence comprend les étapes a), b), c), l'étape c) de traitement (TRAIT) permettant d'obtenir ladite impédance de référence Z0.

6. Procédé selon l'une des revendications 1 à 5, dans lequel la mesure du coefficient de réflexion est mise en oeuvre au moyen d'un analyseur (3) de réseau.

**Patentansprüche**

1. Verfahren zur Überwachung eines Bauwerks (10), das mindestens eine erste metallische Armierung (1) umfasst, die zum mechanischen Halt des Bauwerks beiträgt, und ein Stromkabel (2), das aus mindestens einem Paar elektrischer Leiter gebildet ist, mit einem ersten, elektrisch zugänglichen Ende (21), wobei das Verfahren die folgenden Schritte umfasst:

   a) Messen (MES), durch einen Netzanalysator, des Impedanz-Reflexionskoeffizienten (S11) des Stromkabels in Abhängigkeit von der Frequenz;
   b) Verarbeiten (TRAIT) der ermittelten Reflexionskoeffizienten S11, um daraus die charakteristische Impedanz Zc des Stromkabels in Abhängigkeit von der Distanz abzuleiten, umfassend die folgenden Schritte:

   b1) Extrapolieren der Reflexionskoeffizienten S11 auf eine Null-Frequenz, um ein Signal S11* zu bilden;
   b2) Anwenden eines Anti-Aliasing-Tiefpassfilters auf das Signal S11*;
   b3) Berechnen einer inversen Fourier-Transformation des gefilterten Signals S11* unter Berücksichtigung dessen, dass das Signal S11* nur für Zeiten größer oder gleich 0 existiert und dass eine Lösung der Berechnung der inversen Fourier-Transformation des Signals S11* derart reell ist, dass man eine Impulsantwort des Stromkabels erhält: $h(t) = TF^{-1}(S11^*(f) \times FiltrePB(f))$, wobei $TF^{-1}$ die inverse Fourier-Transformation und $FiltrePB(f)$ der Tiefpassfilter ist;

   b4) Integrieren der Impulsantwort, um eine Antwort auf einer Ebene $u(t) = \int_0^t h(\tau)d\tau$ zu erhalten; und
   b5) Anwenden einer verfeinerten Konversion auf die Antwort auf einer Ebene, um die Antwort auf einer Ebene in eine Impedanz umzuwandeln: $Z_c(t) = Z_{VNA}\frac{1+u(t)}{1-u(t)}$, mit $Z_{VNA}$ als eine interne Impedanz des Netzanalysators;

   c) Vergleichen (COMP) der charakteristischen Impedanz mit einer Referenzimpedanz Z0, die einem Referenzzustand des Stromkabels entspricht, um daraus eine von einer charakteristischen physikalischen Größe des Kabels abhängige Impedanzschwankung $\Delta Z$ entlang des Stromkabels abzuleiten.

2. Verfahren nach Anspruch 1, wobei die charakteristische physikalische Größe des Kabels eine Längsverformung $\varepsilon_Z$ des Stromkabels ist, wobei die Impedanzschwankung $\Delta Z$ durch $\Delta Z = C_\varepsilon \times \varepsilon_z$ definiert ist, mit $C_\varepsilon$ als Verformungssensibilität des Stromkabels, die vorzugsweise von den geometrischen Eigenschaften und den Materialien des Stromkabels (2) abhängt.

3. Verfahren nach Anspruch 1, wobei die charakteristische physikalische Größe des Stromkabels eine vom Temperatur- oder Radialdrucksensibilitätskoeffizienten abhängige Temperaturschwankung ist, wobei die Impedanzschwankung $\Delta Z$ durch $\Delta Z = C_t \times \Delta t$ mit $C_t$ als Temperatursensibilität und $\Delta t$ als Temperaturschwankung definiert ist.

4. Verfahren nach einem der Ansprüche 1 bis 3, das einen Schritt des Bestimmens einer Referenzimpedanz des Stromkabels (2) umfasst, wobei das Stromkabel in diesem Referenzzustand einzeln und unabhängig vom zu überwachenden Bauwerk (10) herangezogen wird.

5. Verfahren nach Anspruch 4, wobei die Bestimmung der Referenzimpedanz die Schritte a), b), c) umfasst, wobei der Schritt c) der Verarbeitung (TRAIT) erlaubt, die Referenzimpedanz Z0 zu erhalten.

6. Verfahren nach einem der Ansprüche 1 bis 5, wobei die Messung des Reflexionskoeffizienten mit einem Netzanalysator (3) durchgeführt wird.

**Claims**

1. A method for monitoring a civil engineering structure (10) comprising at least a first metal reinforcement (1) contributing to the mechanical strength of said structure, and an electric cable (2) consisting of at least one pair of electrical conductors having a first electrically accessible end (21), the method comprising the following steps:

   a) measurement (MES), by a network analyser, of the impedance reflection coefficient (S11) of the electric cable as a function of frequency;

b) processing (TRAIT) the recovered reflection coefficients S11 to derive the characteristic impedance Zc of the electric cable as a function of distance comprising the following steps:

> b1) extrapolate the reflection coefficients S11 to a zero frequency to form a signal S11* ;
>
> b2) apply an anti-aliasing low-pass filter to the signal 511*;
>
> b3) calculate an inverse Fourier transform of the filtered signal S11* considering that the signal S11* exists only for times greater than or equal to 0 and that a solution of the calculation of the inverse Fourier transform of the signal S11* is real, so as to obtain an impulse response of the electric cable : $h(t) = TF^{-1}(S11^*(f) \times FiltrePB(f))$ with $TF^{-1}$ the inverse Fourier transform and $FiltrePB(f)$ the low-pass filter;
>
> b4) integrating the impulse response, to obtain a step response $u(t) = \int_0^t h(\tau)d\tau$ ; and
>
> b5) apply an affine conversion to the step response to transform the step response into an impedance :
>
> $$Z_c(t) = Z_{VNA}\frac{1+u(t)}{1-u(t)}$$ with $Z_{VNA}$ an internal impedance of the network analyser ;

c) comparison (COMP) of the characteristic impedance with a reference impedance Z0 corresponding to a reference state of the electric cable so as to deduce an impedance variation $\Delta Z$ along the electric cable as a function of a characteristic physical quantity of the cable.

2.  A method according to claim 1, wherein the characteristic physical quantity of the cable is a longitudinal deformation $\varepsilon_z$ of the electric cable, the change in impedance $\Delta Z$ being defined by $\Delta Z = C_\varepsilon \times \varepsilon_z$ with $C_\varepsilon$ the sensitivity to the deformation of the electric cable, which preferably depends on the geometrical characteristics and materials of the electric cable (2).

3.  A method according to claim 1, wherein the characteristic physical quantity of the electric cable is a temperature variation depending on the temperature sensitivity coefficient or a radial pressure, the impedance variation $\Delta Z$ being defined by $\Delta Z = C_t \times \Delta t$ with $C_t$ the temperature sensitivity and $\Delta t$ the temperature variation.

4.  A method according to any of claims 1 to 3, comprising a step of determining a reference impedance of the electric cable (2), said electric cable, in this reference state, being taken in isolation and independently of the civil engineering structure (10) to be monitored.

5.  A method according to claim 4, wherein the determination of the reference impedance comprises steps a), b), c), the processing step c) (TRAIT) obtaining said reference impedance Z0.

6.  A method according to any of claims 1 to 5, wherein the measurement of the reflection coefficient is implemented by means of a network analyser (3).

## FIG. 1

## FIG. 2

## FIG. 3

21  22  23  24

## FIG. 4

EP 3 548 879 B1

FIG. 5a

Log (Abs(S11)) (dB)

F (MHz)

FIG. 5b

Phase (S11) (rad)

F (MHz)

FIG. 6

EP 3 548 879 B1

FIG. 7

EP 3 548 879 B1

FIG. 8

EP 3 548 879 B1

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **MARK W. LIN et al.** Crack damage détection of concrète structures using distributed electrical time domain reflectometry (ETDR) sensors. *SPIE - INTERNATIONAL SOCIETY FOR OPTICAL ENGINEERING,* 18 Mai 1999, vol. 3671, 297-304

- **SHISHUANG SUN et al.** A Novel TDR-Based Coaxial Cable Sensor for Crack/Strain Sensing in Reinforced Concrete Structures. *IEEE TRANSACTIONS ON INSTRUMENTATION AND MEASUREMENT,* 01 Août 2009, vol. 58 (8), 2714-2725
- **WEI LIU et al.** *An Overview of Corrosion Damage Détection in Steel Bridge Strands Using TDR,* 31 Décembre 2001, 1-9